# EUROPEAN PATENT SPECIFICATION

(11) **EP 3 171 864 B1**
(45) Date of publication and mention of the grant of the patent: **25.11.2020**
(21) Application number: 15748308.2
(22) Date of filing: 15.07.2015
(51) Int. Cl.: A61K 9/51

(54) **NANOPARTICLE FORMULATIONS OF LOW-WATER SOLUBILITY ACTIVES**
NANOPARTIKELFORMULIERUNGEN VON WIRKSTOFFEN MIT GERINGER WASSERLÖSLICHKEIT
FORMULATIONS DE NANOPARTICULES D'AGENTS ACTIFS FAIBLEMENT HYDROSOLUBLES

(30) Priority: 25.07.2014 US 201462029282 P
(43) Date of publication of application: 31.05.2017
(73) Proprietor: Bend Research, Inc., Bend, OR 97701-8599 (US)
(72) Inventor: MILLER, Warren Kenyon, Bend, Oregon 97701 (US); MORGEN, Michael Mark, Bend, Oregon 97701 (US)
(74) Representative: De Clercq & Partners
(86) International application number: PCT/IB2015/055348
(87) International publication number: WO 2016/012906

(56) References cited:
- EP-A1- 2 578 209
- WO-A1-2014/126969
- US-A1- 2009 061 009

## Description

### TECHNICAL FIELD

Disclosed are compositions including nanoparticles comprising a poorly aqueous soluble polymer, an ionizable active agent, and a counterion having an opposite charge as the ionizable active agent, as well as methods of making and using the compositions.

### SUMMARY

Embodiments of compositions including nanoparticles comprising a poorly aqueous soluble polymer, an ionizable active agent, and a counterion having an opposite charge as the ionizable active agent are disclosed. Methods of making and using the compositions are also disclosed.

In one embodiment, a composition comprises nanoparticles comprising (a) a poorly aqueous soluble polymer; (b) an ionizable active agent having a pKa; and (c) a counterion having an opposite charge as the ionizable active agent for said active. The nanoparticles have an average size of less than 1000 nm. The active in said nanoparticles is substantially non-crystalline. The poorly aqueous soluble polymer, the ionizable active agent and the counterion having an opposite charge as the ionizable active agent collectively constitute at least 50 wt% of the nanoparticles. The counterion has a molecular weight of at least 200 Daltons, a Log P value of at least 2, and a pKa that is at least 1.5 units different than the pKa of the active. The molar ratio of the ionizable active agent to said counterion having an opposite charge is at least 0.8.

In some embodiments, the ionizable active agent and the counterion are capable of forming a salt form. In one embodiment, the salt form in pure form has a melt temperature that is 10°C less than the melt temperature of the non-ionized form of the active in pure form. In another embodiment, the salt form in pure form has a melt temperature that is 20°C less than the melt temperature of the non-ionized form of the active in pure form. In any or all of these embodiments, the salt form in pure form may be substantially non-crystalline.

In any or all of the above embodiments, the counterion may have a molecular weight of at least 400 Daltons. In any or all of the above embodiments, the counterion may have a Log P value of at least 3.

In any or all of the above embodiments, the counterion may have a pKa that is at least 2 units different than the pKa of the active agent, or a pKa that is at least 3 units different than the pKa of the active agent. In any or all of the above embodiments, the counterion may comprise at least 8 carbon atoms and at least one ionizable group.

In some embodiments, the counterion comprises at least 8 carbon atoms and at least one acidic group selected from sulfate, sulfonate, phosphate, phosphonate, and carboxylate. In one embodiment, the counterion is 1,4-bis(2-ethylhexoxy)-1,4-dioxobutane-2-sulfonate, cholesteryl sulfate, cholesteryl phosphate, tocopherol sulfate, tocopherol phosphate, 4,4'-methylenebis(3-hydroxy-2-naphthoate), 2,6-di-tert-butylnaphthalene-1-sulfonate, 2,6-di-tert-butylnaphthalene-1,5-disulfoate, 1,4-dicyclohexyloxy-1,4-dioxobutane-2-sulfonate, 1,4-diisopentyloxy-1,4-dioxobutane-2-sulfonate, 2-naphthalene sulfonate, or any combination thereof.

In some embodiments, the counterion comprises at least 8 carbon atoms and at least one cationic group selected from amines. In some embodiments, the cationic group is selected from primary amines, secondary amines, tertiary amines, quaternary amines, pyridines, pyridazines, pyrimidines, imidazoles, pyrroles, quinolines, purines, piperazines, piperidines, pyrazines, pyrazoles, indoles, indolines, guanidines, and mixtures and combinations thereof. In one embodiment, the counterion is cationic, and is benzathine, benethamine, hydrabamine, or a combination thereof.

In any or all of the above embodiments, the poorly aqueous soluble polymer may be cellulose, ethylcellulose (EC), propylcellulose, butylcellulose, cellulose acetate (CA), cellulose propionate, cellulose butyrate, cellulose acetate butyrate (CAB), cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate (HPMCA), dextran acetate, dextran propionate, dextran acetate propionate, polyvinyl caprolactam, polyvinyl acetate, polyoxyethylene castor oils, polycaprolactam, polylactic acid, polyglycolic acid, poly(lactic-glycolic)acid, hydroxypropyl methyl cellulose acetate succinate (HPMCAS), hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1, poly(methacrylic acid-co-methyl methacrylate) 1:2, poly(methacrylic acid-co-ethyl acrylate) 1:1, poloxamers, poly(ethylene oxide-b-ε-caprolactone), poly(ε-caprolactone-b-ethylene glycol), poly(ethylene oxide-b-lactide), poly(lactide-b-ethylene glycol), poly(ethylene oxide-b-glycolide), poly(glycolide-b-ethylene glycol), poly(ethylene oxide-b-lactide-co-glycolide), poly(lactide-co-glycolide-b-ethylene glycol), or any mixture thereof. In one embodiment, the poorly aqueous soluble polymer is cellulose, ethylcellulose (EC), propylcellulose, butylcellulose, cellulose acetate (CA), cellulose propionate, cellulose butyrate, cellulose acetate butyrate (CAB), cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate (HPMCA), dextran acetate, dextran propionate, dextran acetate propionate, polyvinyl caprolactam, polyvinyl acetate, polyoxyethylene castor oils, polycaprolactam, polylactic acid, polyglycolic acid, poly(lactic-glycolic)acid, or any mixture thereof. In another embodiment, the poorly aqueous soluble polymer is ethylcellulose (EC), cellulose acetate (CA), cellulose acetate butyrate (CAB), polylactic acid, polyglycolic acid, poly(lactic-glycolic)acid, or any mixture thereof. In another embodiment, the poorly aqueous soluble polymer is hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1 (e.g., Eudragit® L100, Evonik Industries AG), poly(methacrylic acid-co-methyl methacrylate) 1:2 (e.g., Eudragit® S100), poly(methacrylic acid-co-ethyl acrylate) 1:1 (e.g., Eudragit® L100-55), or any mixture thereof. In another embodiment, the poorly aqueous soluble polymer is a poloxamer, poly(ethylene oxide-b-ε-caprolactone), poly(ε-caprolactone-b-ethylene glycol), poly(ethylene oxide-b-lactide), poly(lactide-b-ethylene glycol), poly(ethylene oxide-b-glycolide), poly(glycolide-b-ethylene glycol), poly(ethylene oxide-b-lactide-co-glycolide), poly(lactide-co-glycolide-b-ethylene glycol), or any mixture thereof.

In any or all of the above embodiments, the molar ratio of the ionizable active agent to the counterion may be at least 1.0. In some embodiments, the molar ratio of the ionizable active agent to the counterion is greater than 1.0.

In any or all of the above embodiments, the nanoparticles may have an average size of less than 500 nm, less than 400 nm, less than 300 nm, or less than 200 nm.

In any or all of the above embodiments, the composition may consist essentially of the poorly aqueous soluble polymer, the ionizable active agent, and the counterion having an opposite charge as the ionizable active agent. In some embodiments, the composition consists of the poorly aqueous soluble polymer, the ionizable active agent, and the counterion having an opposite charge as the ionizable active agent.

A process of making the disclosed compositions includes (a) forming an organic solution comprising an organic solvent, an active agent, a counterion having an opposite charge as the ionizable active agent, and a poorly aqueous soluble polymer; (b) forming an aqueous solution, wherein said active agent, said counterion, and said polymer are poorly soluble in said aqueous solution; (c) mixing said organic solution with said aqueous solution to form a first mixture; and (d) removing at least a portion of said organic solvent from said first mixture to form a suspension comprising said nanoparticles and said aqueous solution, wherein said nanoparticles have an size of less than 1000 nm, and wherein said active agent, said counterion, and said polymer collectively constitute at least 60 wt% of said nanoparticles. In some embodiments, the organic solvent is removed by spray drying, evaporation, extraction, diafiltration, pervaporation, vapor permeation, distillation, filtration, or any combination thereof.

In some embodiments, the process is an emulsification process, and the organic solvent is immiscible with the aqueous solution. In one embodiment, the organic solvent is methylene chloride, trichloroethylene, tetrachloroethane, trichloroethane, dichloroethane, dibromoethane, ethyl acetate, benzyl alcohol, phenol, chloroform, toluene, xylene, ethyl-benzene, methyl-ethyl ketone, methyl-isobutyl ketone, or any mixture thereof. In another embodiment, the organic solvent is methylene chloride, ethyl acetate, benzyl alcohol, or any mixture thereof.

In some embodiments, the process is a precipitation process, and the organic solvent is miscible with the aqueous solution. In one embodiment, the organic solvent is acetone, methanol, ethanol, tetrahydrofuran (THF), dimethylsulfoxide (DMSO), or any mixture thereof.

The disclosed compositions may be formulated into a dosage form. In some embodiments, the dosage form is tablets, capsules, suspensions, powders for suspension, creams, transdermal patches, depots, or any combination thereof. In some embodiments, the dosage form is formulated for administration to an animal via oral, buccal, mucosal, sublingual, intravenous, intra-arterial, intramuscular, subcutaneous, intraperitoneal, intraarticular, infusion, intrathecal, intraurethral, topical, subdermal, transdermal, intranasal, inhalation, pulmonary tract, intratracheal, intraocular, ocular, intraaural, vaginal, or rectal delivery. In one embodiment, the dosage form is formulated for intravenous, intra-arterial, intramuscular, subcutaneous, intraperitoneal, intraarticular, infusion, intrathecal, or intraurethral delivery. In another embodiment, the dosage form is formulated for topical administration, and the formulation is a gel, hydrogel, lotion, liposome, solution, cream, ointment, foam, bandage or microemulsion.

### DETAILED DESCRIPTION

The inventors have found that there is a continuing need to develop methods and compositions to improve the bioavailability of active agents.

The present disclosure relates to compositions including nanoparticles comprising a poorly aqueous soluble polymer, an ionizable active agent, and a counterion having an opposite charge as the ionizable active agent. Various embodiments are disclosed herein. The following description is exemplary in nature and is not intended to limit the scope, applicability, or configuration of the invention in any way. Various changes to the described embodiments may be made in the function and arrangement of the elements described herein without departing from the scope of the invention.

As used in this application and in the claims, the singular forms "a," "an," and "the" include the plural forms unless the context clearly dictates otherwise. Additionally, the term "includes" means "comprises." Further, the term "coupled" generally means electrically, electromagnetically, and/or physically (e.g., mechanically or chemically) coupled or linked and does not exclude the presence of intermediate elements between the coupled or associated items absent specific contrary language.

Unless otherwise indicated, all numbers expressing quantities of ingredients, properties such as molecular weight, percentages, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

As used in this application and in the claims, "pKa" is defined as the negative logarithm of the ionization constant (K) of an acid, which is the pH of a solution in which half of the acid molecules are ionized.

### Active Agents

Embodiments of the disclosed compositions are suitable for use with any biologically active compound desired to be administered to a patient in need of the active agent. The compositions may contain one or more active agents. As used herein, by "active" or "active agent" is meant a drug, medicament, pharmaceutical, therapeutic agent, nutraceutical, or other compound that may be desired to be administered to the body. The active is generally a "small molecule," having a molecular weight of 2000 Daltons or less.

By "ionizable active agent" is meant an active agent that is ionizable in the physiological pH range of the gastro-intestinal tract, such as pH from 1 to 8. An "ionizable active agent" as employed herein is an active that is essentially totally water-insoluble or poorly water-soluble at any pH in the range of pH 2.0 to pH 8.0. In one embodiment, the ionizable active agent has a water solubility of less than 5 mg/mL at any pH in the range of pH 2.0 to pH 8.0. The active agent may have an even lower water solubility at any pH in the range of pH 2.0 to pH 8.0, such as less than 1 mg/mL, less than 0.1 mg/mL, and even less than 0.01 mg/mL.

The active agent should be understood to include the non-ionized form of the active as well as the ionized form of the active. By non-ionized form is meant that the active is not converted to an ionic form. By ionized form is meant that at least one pH in the physiological pH range of 1-8, the active is converted to an ionic form, *i.e.,* a cation or anion. In one embodiment, the active includes pharmaceutically acceptable forms of the active. The term "pharmaceutically acceptable" refers to a substance that can be taken into a subject without significant adverse toxicological effects on the subject. By "pharmaceutically acceptable forms" is meant any pharmaceutically acceptable derivative or variation, including stereoisomers, stereoisomer mixtures, enantiomers, solvates, hydrates, isomorphs, polymorphs, pseudomorphs, neutral forms, and prodrugs of the active.

Examples of classes of active agents include, but are not limited to, compounds for use in the following therapeutic areas: antihypertensives, antianxiety agents, antiarrhythmia agents, anticlotting agents, anticonvulsants, blood glucose-lowering agents, decongestants, antihistamines, antitussives, antineoplastics, anticancer agents, antitumor agents, beta blockers, anti-inflammatories, antipsychotic agents, cognitive enhancers, anti-atherosclerotic agents, cholesterol-reducing agents, triglyceride-reducing agents, antiobesity agents, autoimmune disorder agents, anti-impotence agents, antibacterials, anthelmintics, antihelminthics, antifungal agents, hypnotic agents, anti-Parkinsonism agents, anti-Alzheimer's disease agents, antibiotics, anti-angiogenesis agents, anti-glaucoma agents, anti-depressants, anti-acne agents, bronchodilators, glucocorticoids, steroids, and antiviral agents.

### Counterions

The compositions also include a counterion having an opposite charge as the ionizable active agent. By "counterion" is meant that the counterion has a pKa that is at least 1.5 units different than the pKa of the active. In one embodiment, the pKa of the counterion is at least 2 units different than the pKa of the active. In another embodiment, the pKa of the counterion is at least 3 units different than the pKa of the active.

In one embodiment, the counterion, in its non-ionized state is lipophilic, having an aqueous solubility of less than 1 mg/mL. In other embodiments, the counterion has an aqueous solubility of less than 0.1 mg/mL, less than 0.01 mg/mL, or even less than 0.001 mg/mL.

In one embodiment, the counterion comprises at least 8 carbon atoms, and at least one ionizable group. In another embodiment, the counterion comprises at least 10 carbon atoms and at least one ionizable group. In another embodiment, the counterion comprises at least 12 carbon atoms and at least one ionizable group. In still another embodiment, the counterion comprises at least 14 carbon atoms and at least one ionizable group. In another embodiment, the counterion comprises at least 16 carbon atoms and at least one ionizable group. In still another embodiment, the counterion comprises at least 18 carbon atoms and at least one ionizable group. In one embodiment, the ionizable group is acidic. In another embodiment the ionizable group is acidic and selected from sulfate, sulfonate, phosphate, phosphonate, and carboxylate groups. In still another embodiment, the ionizable group is cationic. In another embodiment the ionizable group is cationic, and selected from amino groups, including primary amines, secondary amines, tertiary amines, quaternary amines, including but not limited to those substituted with aliphatic groups, benzyl groups, heterocyclic bases, including those containing 5 and 6-membered aromatic rings, such as certain pyridines, pyridazines, pyrimidines, imidazoles, pyrroles, quinolines, purines, piperazines, piperidines, pyrazines, pyrazoles, indoles, indolines, guanidines, and mixtures and combinations thereof.

In one embodiment, the ionizable counterion has a pKa that is at least 1.5 units different than the pKa of the active. In another embodiment, the ionizable counterion has a pKa that is at least 2.0 units different than the pKa of the active. In another embodiment, the ionizable counterion has a pKa that is at least 3.0 units different than the pKa of the active.

In one embodiment, the ionizable counterion has a pKa that is at least 1.5 units different than the pKa of the active and a molecular weight that is at least 200 Daltons. In still another embodiment, the ionizable counterion has a pKa that is at least 1.5 units different than the pKa of the active, and a molecular weight that is at least 300 Daltons. In still another embodiment, the ionizable counterion has a pKa that is at least 1.5 units different than the pKa of the active, and a molecular weight that is at least 400 Daltons. In still another embodiment, the ionizable counterion has a pKa that is at least 2 units different than the pKa of the active, and a molecular weight that is at least 200 Daltons. In another embodiment, the ionizable counterion has a pKa that is at least 2 units different than the pKa of the active, and a molecular weight that is at least 300 Daltons. In still another embodiment, the ionizable counterion has a pKa that is at least 2 units different than the pKa of the active, and a molecular weight that is at least 400 Daltons. In still another embodiment, the ionizable counterion has a pKa that is at least 3 units different than the pKa of the active, and a molecular weight that is at least 200 Daltons. In another embodiment, the ionizable counterion has a pKa that is at least 3 units different than the pKa of the active, and a molecular weight that is at least 300 Daltons. In still another embodiment, the ionizable counterion has a pKa that is at least 3 units different than the pKa of the active, and a molecular weight that is at least 400 Daltons.

The active agent and the counterion are capable of forming a salt form. By "capable of forming a salt form" means that the active agent and the counterion having an opposite charge as the ionizable active agent, under the proper conditions, will react to form the corresponding salt form with the active, meaning that ionizable group on the active agent is associated with the counterion having an opposite charge as the active agent. By "associated with" is generally meant that an ion pair is formed between the ionizable counter ion and the active agent.

In another embodiment, the salt form of the ionizable active agent and the counterion having an opposite change as the ionizable active agent has a higher partition coefficient between octanol and water than the ionizable active agent in non-ionized form. By higher partition coefficient is meant that the salt form, when added to an aqueous solution containing octanol, and allowed to come to equilibrium, has a higher equilibrium concentration in the octanol phase as it does in the aqueous phase, relative to the same experiment performed with the active agent in non-ionized form.

In one embodiment, the salt form of the active agent and the counterion in pure form has a melt temperature that is at least 10°C less than the non-ionized form of the active. The melt temperature may be determined using standard techniques, such as modulated differential scanning calorimetry (mDSC), or by a standard melt temperature apparatus. In another embodiment, the salt form of the active agent and the counterion in pure form has a melt temperature that is at least 20°C less than the non-ionized form of the active, or at least 30°C less than the non-ionized form of the active.

In one embodiment, the active, when associated with the counterion, is substantially non-crystalline. By "substantially non-crystalline" is meant that the amount of active in crystalline form does not exceed 20 wt%. In another embodiment, the active, when associated with the counterion, is almost completely non-crystalline, meaning that the amount of active in crystalline form does not exceed 10 wt%. The amount of non-crystalline active in the nanoparticles may be measured by powder X-ray diffraction (PXRD) or modulated differential scanning calorimetry (mDSC), or any other standard quantitative measurement.

In one embodiment, the counterion is selected from 1,4-bis(2-ethylhexoxy)-1,4-dioxobutane-2-sulfonate (also known as docusate), cholesteryl sulfate, cholesteryl phosphate, tocopherol sulfate, tocopherol phosphate, pamoate (4,4'-methylenebis[3-hydroxy-2-naphthoate]), 2,6-di-tert-butylnaphthalene-1-sulfonate, 2,6-di-tert-butylnaphthalene-1,5-disulfonate, 1,4-dicyclohexyloxy-1,4-dioxobutane-2-sulfonate, 1,4-diisopentyloxy-1,4-dioxobutane-2-sulfonate, 2-naphthalene sulfonate, and combinations thereof.

In another embodiment, the counterion is selected from benzathine, benethamine, hydrabamine, and combinations thereof.

In one embodiment, the counterion is 1,4-bis(2-ethylhexoxy)-1,4-dioxobutane-2-sulfonate, having the structure:

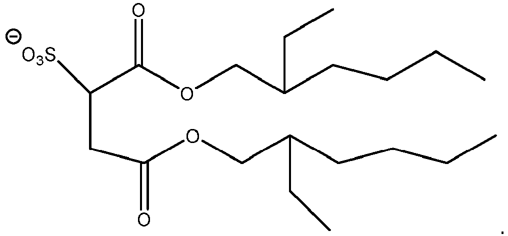

In one embodiment, the counterion is cholesteryl sulfate, having the structure:

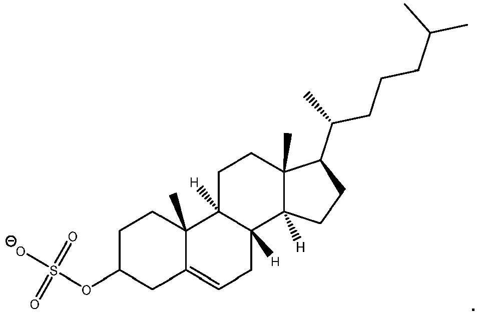

In one embodiment, the counterion is cholesteryl phosphate, having the structure:

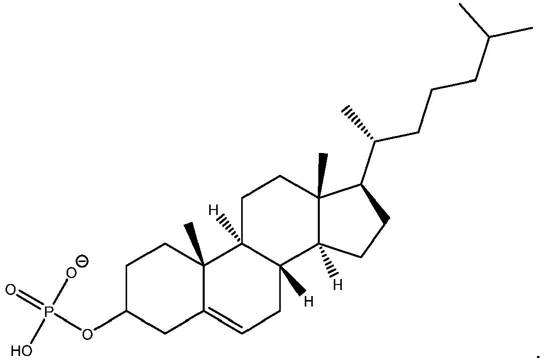

In one embodiment, the counterion is tocopherol sulfate, having the structure:

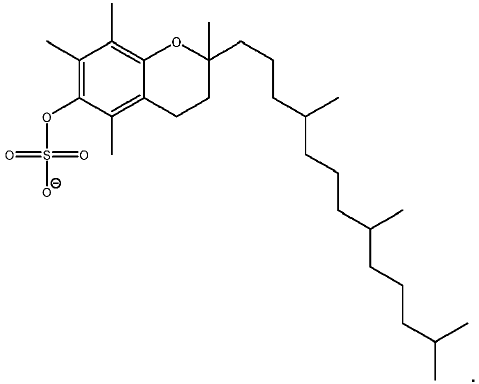

In one embodiment, the counterion is tocopherol phosphate, having the structure:

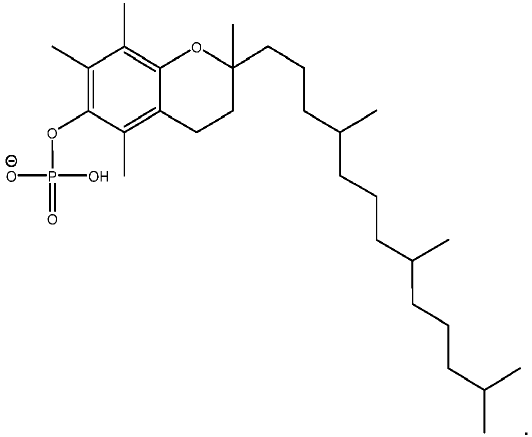

In one embodiment, the counterion is pamoate, having the structure:

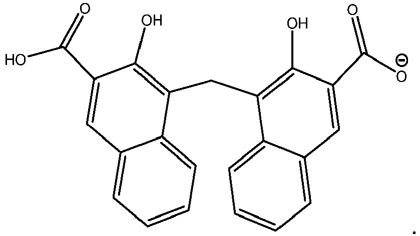

In one embodiment, the counterion is 2,6-di-tert-butylnaphthalene-1-sulfonate, having the structure:

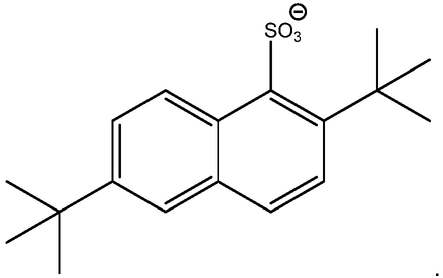

In one embodiment, the counterion is 2,6-di-tert-butylnaphthalene-1,5-disulfonate, having the structure:

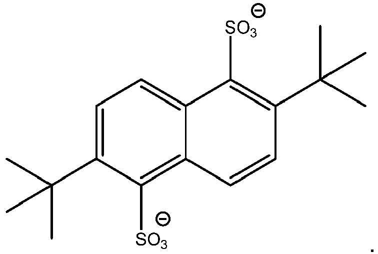

In one embodiment, the counterion is 1,4-dicyclohexyloxy-1,4-dioxobutane-2-sulfonate, having the structure:

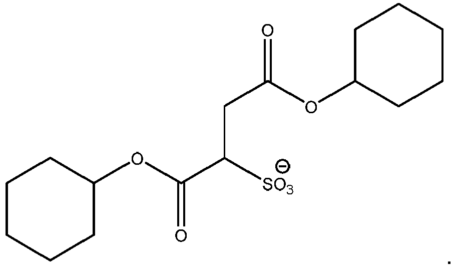

In one embodiment, the counterion is 1,4-diisopentyloxy-1,4-dioxobutane-2-sulfonate, having the structure:

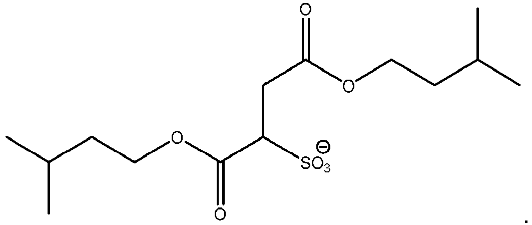

In one embodiment, the counterion is 2-naphthalene sulfonate, having the structure:

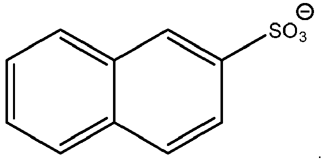

In one embodiment, the counterion is N,N'-dibenzylethylene diamine (also known as benzathine), having the structure:

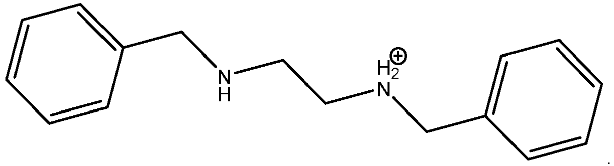

In one embodiment, the counterion is N-(benzyl)-2-phenylethan-1-amine (also known as benethamine), having the structure:

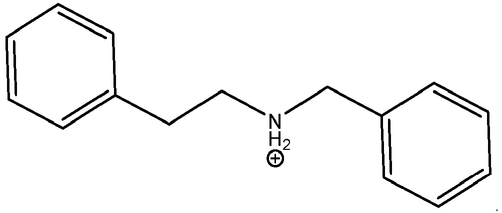

In one embodiment, the counterion is N¹,N²-bis(((1R,4aS,10aR)-7-isopropyl-1,4a-dimethyl-1 ,2,3,4,4a,9,10,10a-octahydrophenanthren-1-yl)methyl)ethane-1,2-diamine (also known as hydrabamine), having the structure:

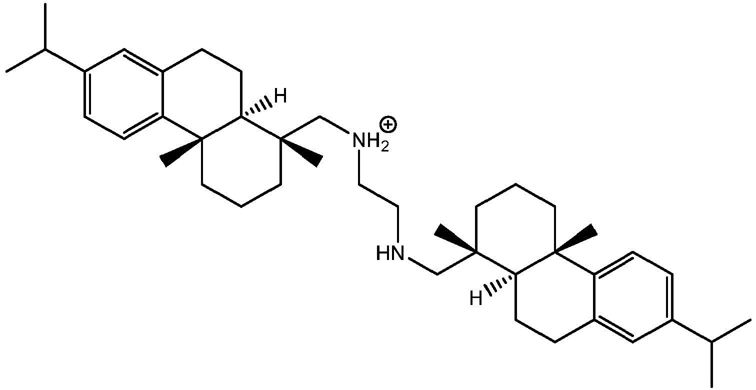

Table 1 lists some of the properties of the counterions.

**Table 1. Properties of Counterions.**

| Counterion | Formula | Molecular Weight (Daltons) | pKa | Log P (Calculated) |
|---|---|---|---|---|
| 1,4-bis(2-ethylhexoxy)-1,4- dioxobutane-2-sulfonate | C₂₀H₃₈O₇S | 422.5 | <1 estimated | 4.84 |
| Cholesteryl sulfate | C₂₇ H₄₆ O₄S | 466.7 | <1 estimated | >4 estimated |
| Cholesteryl phosphate | C₂₇H₄₆O₄P | 466.6 | < 2 estimated | > 7 estimated |
| Tocopherol phosphate | C₂₉HOP | 510.6 | <2 estimated | 10.0 |
| Tocopherol sulfate | C₂₉H₅₀O₅S | 510.8 | <1 estimated | 9.1 |
| 4,4'-methylenebis(3-hydroxy-2-naphthoate) (pamoate) | C₂₃H₁₆O₆ | 388 | 2.5, 3.1 | 4.46 |
| 2,6-di-tert-butylnaphthalene-1-sulfonate | C₁₈H₂₄O₃S | 320 | -2.48 | 5.6 |
| 2,6-di-tert-butylnaphthalene-1,5-disulfonate | C₁₈H₂₄O₆S₂ | 400.5 | -2.32, -3.05 | 4.76 |
| 1,4-dicyclohexyloxy-1,4-dioxobutane-2-sulfonate | C₁₆H₂₆O₇S | 362.4 | <1 estimated | 2.13 |
| 1,4-diisopentyloxy-1,4-dioxobutane-2-sulfonate | C₁₄H₂₆O₇S | 338.4 | <1 estimated | 2.18 |
| 2-Naphthalenesulfonate | C₁₀H₈O₃S | 208.2 | 0.17 | 2.17 |
| N,N'-dibenzylethylene diamine | C₁₆H₂₀N₂ | 240.3 | 6.2 | 3.2 |
| N-(benzyl)-2-phenylethan-1-amine | C₁₅H₁₇N | 211.3 | 9.4 | 3.6 |
| N',N²-bis(((1R,4aS,10aR)-7-isopropyl-1,4a-dimethyl-1,2,3,4,4a,9,10,10a-octahydrophenanthren-1-yl)methyl)ethane-1,2-diamine | C₄₂H₆₄N₂ | 597.0 | 6.6 | 13.2 |

In one embodiment, the molar ratio of ionizable active agent to counterion having an opposite charge is at least 0.8. In another embodiment, the molar ratio of ionizable active agent to counterion having an opposite charge is at least 0.9. In still another embodiment, the molar ratio of ionizable active agent to counterion having an opposite charge is at least 1.0. In another embodiment, the molar ratio of ionizable active agent to counterion having an opposite charge is greater than 1.0.

In one embodiment, molar ratio of the ionizable active agent to counterion having an opposite charge is greater than 1.0 . The inventors have found that including such a formulation can improve the performance when the composition is administered to an aqueous use environment, such as *in vivo* and *in vitro* use environments.

In one embodiment, the ionizable active agent and counterion having an opposite charge are combined during processing to form the drug product, and the salt form is never isolated.

### Poorly Aqueous Soluble Polymers

Embodiments of the disclosed compositions comprise at least one poorly aqueous soluble polymer. By "poorly aqueous soluble" is meant that the polymer has a solubility of less than 10 mg/mL when administered alone at a concentration of from 50 to 100 mg/mL to a buffered solution at a physiologically relevant pH range of pH 1-8. A test to determine whether the polymer is poorly aqueous may be performed as follows. The polymer is initially present in bulk powder form with average particle sizes of greater than 1 micron. The polymer alone is administered to the buffer solution and stirred for approximately 1 hour at room temperature. Next, a nylon 0.45 µm filter is weighed, and the solution is filtered. The filter is dried overnight at 40°C, and weighed the following morning. The amount of polymer dissolved is calculated from the amount of polymer added to the buffer solution minus the amount of polymer remaining on the filter (mg). The polymer is considered to be poorly aqueous soluble if it has a solubility of less than 10 mg/mL in this test. In other embodiments, the polymer is considered poorly aqueous soluble if it has a solubility of less than 5 mg/mL in this test, less than 1 mg/mL in this test, or even less than 0.1 mg/mL in this test.

Exemplary polymers include cellulose, ethylcellulose (EC), propylcellulose, butylcellulose, cellulose acetate (CA), cellulose propionate, cellulose butyrate, cellulose acetate butyrate (CAB), cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate (HPMCA), dextran acetate, dextran propionate, dextran acetate propionate, polyvinyl caprolactam, polyvinyl acetate, polyoxyethylene castor oils, polycaprolactam, polylactic acid, polyglycolic acid, poly(lactic-glycolic)acid, hydroxypropyl methyl cellulose acetate succinate (HPMCAS), hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1 (e.g., Eudragit® L100, Evonik Industries AG), poly(methacrylic acid-co-methyl methacrylate) 1:2 (e.g., Eudragit® S100), poly(methacrylic acid-co-ethyl acrylate) 1:1 (e.g., Eudragit® L100-55), poloxamers, poly(ethylene oxide-b-ε-caprolactone), poly(ε-caprolactone-b-ethylene glycol), poly(ethylene oxide-b-lactide), poly(lactide-b-ethylene glycol), poly(ethylene oxide-b-glycolide), poly(glycolide-b-ethylene glycol), poly(ethylene oxide-b-lactide-co-glycolide), poly(lactide-co-glycolide-b-ethylene glycol), and mixtures thereof.

In one embodiment, the poorly aqueous soluble polymer is selected from ethylcellulose (EC), propylcellulose, butylcellulose, cellulose acetate (CA), cellulose propionate, cellulose butyrate, cellulose acetate butyrate (CAB), cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate (HPMCA), dextran acetate, dextran propionate, dextran acetate propionate, polyvinyl caprolactam, polyvinyl acetate, polyoxyethylene castor oils, polycaprolactam, polylactic acid, polyglycolic acid, poly(lactic-glycolic)acid, and mixtures thereof. In another embodiment, the poorly aqueous soluble polymer is selected from ethylcellulose (EC), cellulose acetate (CA), cellulose acetate butyrate (CAB), polylactic acid, polyglycolic acid, poly(lactic-glycolic)acid, and mixtures thereof.

In another embodiment, the poorly aqueous soluble polymer has a pH-dependent solubility profile. In one embodiment, the poorly aqueous soluble polymer is an enteric polymer, having a low aqueous solubility at pH levels below 6. Exemplary enteric polymers include hydroxypropyl methyl cellulose acetate succinate (HPMCAS), hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1 (e.g., Eudragit® L100, Evonik Industries AG), poly(methacrylic acid-co-methyl methacrylate) 1:2 (e.g., Eudragit® S100), poly(methacrylic acid-co-ethyl acrylate) 1:1 (e.g., Eudragit® L100-55), and mixtures thereof.

In another embodiment, the poorly aqueous soluble polymer is capable of forming micelles at a low concentration in water. By "low concentration" is meant a concentration of less than 5 wt% in water, such as a concentration of less than 4 wt%, less than 3 wt%, or less than 2 wt%. By "micelles" is meant an aggregate of molecules present as a colloid or nanoparticle of less than 100 nm in diameter. When poorly aqueous soluble polymers are added to an aqueous solution, there is typically a concentration above which the polymer molecules aggregate. These aggregates can be called micelles or nanoparticles, and they can be analyzed by light scattering analysis, such as dynamic light scattering (DLS) techniques, turbidity measurements, or imaging techniques such as electron microscopy. Exemplary poorly aqueous soluble polymers that form micelles include poloxamers, poly(ethylene oxide-b-ε-caprolactone), poly(ε-caprolactone-b-ethylene glycol), poly(ethylene oxide-b-lactide), poly(lactide-b-ethylene glycol), poly(ethylene oxide-b-glycolide), poly(glycolide-b-ethylene glycol), poly(ethylene oxide-b-lactide-co-glycolide), poly(lactide-co-glycolide-b-ethylene glycol), and mixtures thereof.

### Compositions

Embodiments of the disclosed compositions comprise nanoparticles comprising a poorly aqueous soluble polymer, an ionizable active agent, and a counterion having an opposite charge as the ionizable active agent. In one embodiment, the poorly aqueous soluble polymer, the ionizable active agent, and the counterion having an opposite charge as the ionizable active agent collectively constitute at least 50 wt% of the nanoparticles. In another embodiment, the poorly aqueous soluble polymer, an ionizable active agent, and a counterion having an opposite charge as the ionizable active agent collectively constitute at least 80 wt% of the nanoparticles. In still another embodiment, the poorly aqueous soluble polymer, an ionizable active agent, and a counterion having an opposite charge as the ionizable active agent collectively constitute at least 90 wt% of the nanoparticles. In another embodiment, the composition consists essentially of the poorly aqueous soluble polymer, the ionizable active agent, and the counterion having an opposite charge as the ionizable active agent. As used herein, "consists essentially of" means that the composition may include small amounts (*i.e.,* less than 5 wt%) of other components that do not materially alter the composition, such as other excipients. In another embodiment, the composition consists of the poorly aqueous soluble polymer, the ionizable active agent, and the counterion having an opposite charge as the ionizable active agent.

The nanoparticles are small particles comprising a poorly aqueous soluble polymer, an ionizable active agent, and a counterion having an opposite charge as the ionizable active agent. By "nanoparticles" is meant a plurality of small particles in which the average size of the particles is less than 1000 nm. In suspension, by "size" is meant the effective cumulant diameter as measured by dynamic light scattering (DLS), using for example, Brookhaven Instruments' 90Plus particle sizing instrument. By "size" is meant the diameter if the particles were spherical particles, or the maximum diameter for non-spherical particles. In one embodiment, the average size of the nanoparticles is less than 500 nm, less than 400 nm, less than 300 nm, less than 200 nm, less than 150 nm, or even less than 100 nm.

In one embodiment, the disclosed compositions consist essentially of a poorly aqueous soluble polymer, an ionizable active agent, and a counterion having an opposite charge as the ionizable active agent. In other embodiments, the disclosed compositions consist of a poorly aqueous soluble polymer, an ionizable active agent, and a counterion having an opposite charge as the ionizable active agent.

In one embodiment, the amount of ionizable active agent in the nanoparticles may range from 0.01 wt% to 99 wt% active. In other embodiments, the amount of active may range from 0.1 wt% to 80 wt%, or from 0.1 to 60 wt%, or from 1 to 40 wt%. In other embodiments, the amount of active in the nanoparticles may be at least 1 wt%, at least 5 wt%, at least 10 wt%, at least 15 wt%, at least 20 wt%, at least 25 wt%, at least 30 wt%, at least 40 wt%, at least 45 wt%, or even at least 50 wt%.

In one embodiment, the active is present in the nanoparticles as a salt form of the active and the counterion.

In one embodiment, the active in the nanoparticles (or the salt form of the active and the counterion) is present in a non-crystalline state. In one embodiment, the non-crystalline active can exist as a pure active phase, as a solid solution of active homogeneously distributed throughout the polymer, or any combination of these states or those that lie intermediate between them. A "solid solution" is formed when at least one solid component is molecularly dispersed within another solid component, resulting in a homogeneous or substantially homogeneous solid material. In another embodiment, at least 80 wt% of the active in the nanoparticles is present in a non-crystalline state. In another embodiment, at least 90 wt% of the active in the nanoparticles is present in a non-crystalline state. In still another embodiment, essentially all of the active in the nanoparticles is in a non-crystalline state. In one embodiment, the non-crystalline active is present as a solid solution of active homogeneously distributed throughout the polymer.

The amount of non-crystalline active in the nanoparticles may be measured by powder X-ray diffraction (PXRD) or modulated differential scanning calorimetry (mDSC), or any other standard quantitative measurement.

### Methods for Forming Nanoparticles

In one method of forming embodiments of the disclosed compositions, the ionizable active agent, the counterion having an opposite charge as the ionizable active agent, and a poorly aqueous soluble polymer are mixed with a solvent to form a liquid solution or liquid suspension. The nanoparticles may then be formed from the liquid solution or suspension by any known process, including precipitation in a miscible non-solvent, emulsifying in an immiscible non-solvent, or by forming droplets followed by removal of the solvent by evaporation to produce particles.

The nanoparticles may be formed by any process that results in formation of nanoparticles of an ionizable active agent, a counterion having an opposite charge as the ionizable active agent, and a poorly aqueous soluble polymer. One process for forming nanoparticles is an emulsification process. In this process, the ionizable active agent, counterion, and poorly aqueous soluble polymer are dissolved in an organic solvent that is immiscible with an aqueous solution in which the active agent, counterion, and polymer are poorly soluble, forming an organic solution. Solvents suitable for forming the organic solution of dissolved active agent, counterion, and polymer can be any compound or mixture of compounds in which the active agent, counterion, and the polymer are mutually soluble and which is immiscible with the aqueous solution. As used herein, the term "immiscible" means that the organic solvent has a solubility in the aqueous solution of less than 20 wt%, or less than 10 wt%, or less than 5 wt%, or even less than 3 wt%. In one embodiment, the organic solvent is also volatile with a boiling point of 150 °C or less. Exemplary solvents include methylene chloride, trichloroethylene, tetrachloroethane, trichloroethane, dichloroethane, dibromoethane, ethyl acetate, phenol, chloroform, toluene, xylene, ethyl-benzene, methyl-ethyl ketone, methyl-isobutyl ketone, and mixtures thereof. In one embodiment, the organic solvent is methylene chloride, ethyl acetate, benzyl alcohol, or a mixture thereof. In one embodiment, the aqueous solution is water.

Once the organic solution is formed, it is then mixed with the aqueous solution and mixed at high shear, for example via homogenization, to form an emulsion of fine droplets of the water immiscible solvent distributed throughout the aqueous phase. The volume ratio of organic solution to aqueous solution used in the process will generally range from 1:100 (organic solution:aqueous solution) to 2:3 (organic solution:aqueous solution). In one embodiment, the organic solution:aqueous solution volume ratio ranges from 1:9 to 1:2 (organic solution:aqueous solution). The emulsion is generally formed by a two-step homogenization procedure. The solution of active agent, counterion, polymer and organic solvent are first mixed with the aqueous solution using a rotor/stator or similar mixer to create a "pre-emulsion". This mixture is then further processed with a high-pressure homogenizer that subjects the droplets to very high shear, creating a uniform emulsion of very small droplets. A portion of the solvent is then removed such that the droplets solidify forming a suspension of the nanoparticles in the aqueous solution. Exemplary processes for removing the organic solvent include spray drying, evaporation, extraction, diafiltration, pervaporation, vapor permeation, distillation, filtration, and combinations thereof. In one embodiment, the solvent is removed to a level that is acceptable according to The International Committee on Harmonization (ICH) guidelines. In one embodiment, the concentration of solvent in the nanoparticle suspension is less than the solubility of the solvent in the aqueous solution. Even lower concentrations of solvent may be obtained. Thus, the concentration of solvent remaining in the nanoparticle suspension following the removal steps may be less than 5 wt%, less than 3 wt%, less 1 wt%, and even less than 0.1 wt%.

An alternative process to form the nanoparticles is a precipitation process. In this process, the active agent, counterion, and polymers are first dissolved in an organic solvent that is miscible with an aqueous solution in which the active agent, counterion, and polymers are poorly soluble to form an organic solution. The organic solution is mixed with the aqueous solution causing the nanoparticles to precipitate. Solvents suitable for forming the organic solution of dissolved active agent, counterion, and polymers can be any compound or mixture of compounds in which the active agent, counterion, and the polymer are mutually soluble and which is miscible with the aqueous solution. In one embodiment, the organic solvent is also volatile with a boiling point of 150°C or less. In addition, the organic solvent should have relatively low toxicity. Exemplary solvents include acetone, methanol, ethanol, tetrahydrofuran (THF), dimethylsulfoxide (DMSO), and ethyl acetate (as long as the aqueous:organic volume ratio is greater than 15). Mixtures of solvents, such as 50% methanol and 50% acetone, can also be used, so long as the active agent, counterions, and polymers, are sufficiently soluble to dissolve the active agent, counterion, and polymers. In one embodiment, the solvent is methanol, acetone, or a mixture thereof.

The aqueous solution may be any compound or mixture of compounds in which the active agent, counterion, and polymers are sufficiently insoluble so as to precipitate to form nanoparticles. In one embodiment, the aqueous solution is water. The water may contain additives, such as acid, base, or buffers to control the pH, surfactants such as sodium glycocholate to control the interfacial tension or salts to control the ionic strength of the aqueous solution.

The organic solution and aqueous solution are combined under conditions that cause solids to precipitate as nanoparticles. The mixing can be by addition of a bolus or stream of organic solution to a stirring container of the aqueous solution. Alternately a stream or jet of organic solution can be mixed with a moving stream of aqueous solution. In either case, the precipitation results in the formation of a suspension of nanoparticles in the aqueous solution.

For the precipitation process, the amount of active agent, counterion and polymers in the organic solution depends on the solubility of each in the organic solvent and the desired ratios of active agent and counterion to polymers in the resulting nanoparticles. The organic solution may comprise from 0.1 wt% to 20 wt% dissolved solids, such as a dissolved solids content of from 0.5 wt% to 10 wt%.

The organic solution:aqueous solution volume ratio should be selected such that there is sufficient aqueous solution in the nanoparticle suspension that the nanoparticles solidify and do not rapidly agglomerate. However, too much aqueous solution will result in a very dilute suspension of nanoparticles, which may require further processing to concentrate the nanoparticles for ultimate use. Generally, the organic solution:aqueous solution volume ratio is at least 1:100, but generally is less than 1:2 (organic solution:aqueous solution). In one embodiment, the organic solution:aqueous solution volume ratio ranges from 1:20 to 1:3.

Once the nanoparticle suspension is made, a portion of the organic solvent may be removed from the suspension using methods known in the art. Exemplary processes for removing the organic solvent include evaporation, spray drying, extraction, diafiltration, pervaporation, vapor permeation, distillation, filtration, and combinations thereof. In one embodiment, the solvent is removed to a level that is acceptable according to ICH guidelines. Thus, the concentration of solvent in the nanoparticle suspension may be less than 10 wt%, less than 5 wt%, less than 3 wt%, less than 1 wt%, and even less than 0.1 wt%.

Both the emulsion process and the precipitation process result in the formation of a suspension of the nanoparticles in the aqueous solution. In some instances it is desirable to concentrate the nanoparticles or to isolate the nanoparticles in solid form by removing some or all of the liquid from the suspension. Exemplary processes for removing at least a portion of the liquid include spray drying, spray coating, spray layering, lyophylization, evaporation, vacuum evaporation, filtration, ultrafiltration, reverse osmosis, and other processes known in the art. In one embodiment, the process is spray drying. In one embodiment, the process is lyophilization. One or more processes may be combined to remove the liquid from the nanoparticle suspension to yield a solid composition. For example, a portion of the liquids may be removed by filtration to concentrate the nanoparticles, followed by spray-drying to remove most of the remaining liquids, followed by a further drying step such as tray-drying. Alternatively, the liquids may be removed by lyophilization.

### Dry Powder Formulations

In certain embodiments, the disclosed compositions are in the form of particles of a solid dry powder, substantially all particles comprising a plurality of nanoparticles. As used herein, the term "particles" means small pieces of matter having characteristic diameters of less than 3000 µm. In another embodiment, the particles are granulated into granules using standard methods known in the art, such as dry granulation, wet granulation, high shear granulation, and the like.

In one embodiment, the mean size of the particles is less than 500 µm. In another embodiment, the mean size of the particles is less than 200 µm. In still another embodiment, the mean size of the particles is less than 100 µm. In one embodiment, the mean size of the particles ranges from 0.5 to 500 µm. In another embodiment, the mean size of the particles ranges from 0.5 to 200 µm. In one embodiment, the mean size of the particles ranges from 0.5 to 100 µm. In one embodiment, the mean size of the particles ranges from 10 to 100 µm. In one embodiment, the mean size of the particles ranges from 10 to 70 µm. In one embodiment, the mean size of the particles ranges from 10 to 50 µm. In one embodiment, the mean size of the particles ranges from 0.5 to 10 µm. In one embodiment, the mean size of the particles ranges from 0.5 to 7 µm.

In one embodiment, the salt form of the ionizable active agent with the counterion having an opposite charge as the ionizable active agent may be made and isolated, followed by forming a composition as disclosed herein. In another embodiment, the salt form of the active and the counterion may be formed *in situ* in an appropriate solvent, and subsequently formed into a composition as disclosed herein.

When isolating the nanoparticles in solid form, it is often desirable to include a matrix material in the suspension of nanoparticles prior to removal of the liquids. The matrix material functions to help slow or prevent agglomeration of the nanoparticles as the liquids are being removed, as well as to help re-suspend the nanoparticles when the solid composition is added to an aqueous solution (e.g., an aqueous environment of use). In one embodiment, the matrix material is pharmaceutically acceptable and water soluble. Examples of matrix materials include polyvinyl pyrrolidone (PVP), trehalose, hydroxypropyl methyl cellulose (HPMC), hydroxypropyl cellulose (HPC), carboxymethyl ethylcellulose (CMEC), hydroxypropyl methylcellulose acetate succinate (HPMCAS), casein, caseinate, albumin, gelatin, acacia, lactose, mannitol, pharmaceutically acceptable forms thereof, and other matrix materials known in the art.

### Methods of Use

In one embodiment, compositions comprising the disclosed nanoparticles can be formulated into suitable dosage forms, such as tablets, capsules, suspensions, powders for suspension, creams, transdermal patches, depots, and the like. Conventional formulation excipients may be employed in embodiments of the disclosed compositions, including those excipients well-known in the art, such as those described in Remington: The Science and Practice of Pharmacy (20th ed., 2000). Generally, excipients such as fillers, disintegrating agents, pigments, binders, lubricants, glidants, flavorants, and so forth may be used for customary purposes and in typical amounts without adversely affecting the properties of the compositions.

In one embodiment, the disclosed compositions are intended for administration to an animal via a mode selected from oral, buccal, mucosal, sublingual, intravenous, intra-arterial, intramuscular, subcutaneous, intraperitoneal, intraarticular, infusion, intrathecal, intraurethral, topical, subdermal, transdermal, intranasal, inhalation, pulmonary tract, intratracheal, intraocular, ocular, intraaural, vaginal, and rectal.

When intended for oral, buccal, mucosal, or sublingual administration, the ionizable active agent, the counterion, and the poorly aqueous soluble polymer are formulated into appropriate dosage forms.

For intravenous, intra-arterial, intramuscular, subcutaneous, intraperitoneal, intraarticular, infusion, intrathecal, or intraurethral delivery, the ionizable active agent, the counterion, and a poorly aqueous soluble polymer are formulated into appropriate dosage forms.

For topical administration, the ionizable active agent, the counterion, and the polymer are formulated into appropriate formulations including gels, hydrogels, lotions, liposomes, solutions, creams, ointments, foams, bandages and microemulsions. Typical carriers for such formulations include alcohol, water, mineral oil, liquid petrolatum, white petrolatum, glycerin, polyethylene glycols, and propylene glycols.

### EXAMPLES

### Active Agents

Erlotinib, also known as N-(3-ethynylphenyl)-6,7-bis(2-methoxyethoxy) quinazolin-4-amine, having the following structure, was used in the Examples.

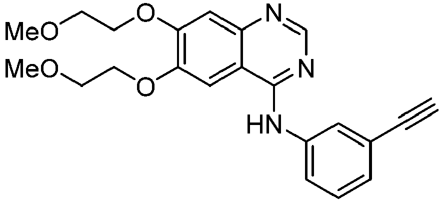

Erlotinib has a melt temperature of 157°C, and a glass-transition temperature of 37°C. The Log P value is 2.7, and it has a pKa of 5.42. Erlotinib has a solubility in phosphate buffered saline (pH 6.5) of 1 µg/mL.

Adapalene, also known as 6-3-(adamantan-1-yl)-4-methoxyphenyl)-2-naphthoic acid, having the following structure, was used in the Examples.

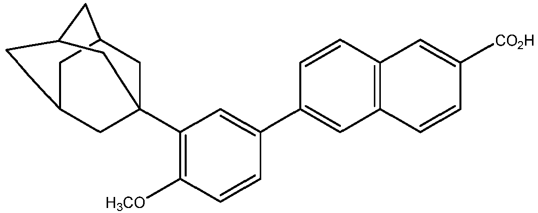

Adapalene has a melt temperature of 320°C. The Log P value is 6.6, and it has a pKa of 4.2.

### Example 1

A 0.2275 g sample of sodium 1,4-bis(2-ethylhexoxy)-1,4-dioxobutane-2-sulfonate was placed in a 20 mL glass vessel with 10 mL tetrahydrofuran (THF) and stirred with a magnetic stir bar until completely dissolved. A 0.41 mL sample of 1.25 M HCl in methanol was added with stirring yielding a white precipitate. The mixture was chilled to 0°C to ensure complete precipitation of sodium chloride. The mixture was then filtered through a 0.2 micron filter to remove sodium chloride followed by an additional rinse of 10 mL THF to extract all of the 1,4-bis(2-ethylhexoxy)-1,4-dioxobutane-2-sulfonate, having a pKa of less than 1. The solution was placed on a rotoevaporator and solvents were removed resulting in a clear film of 1,4-bis(2-ethylhexoxy)-1,4-dioxobutane-2-sulfonate in its protonated (non-ionized) form. The entire sample of the protonated 1,4-bis(2-ethylhexoxy)-1,4-dioxobutane-2-sulfonate was dissolved in 10 mL of THF.

A 0.200 mg sample of Erlotinib free base, having a pKa of 5.4 was dissolved in 10 mL THF. The difference between the pKa of Erlotinib free base with the counterion 1,4-bis(2-ethylhexoxy)-1,4-dioxobutane-2-sulfonate was at least 4.4. The resulting clear solution was stirred for several minutes. This erlotinib solution was added to the sodium 1,4-bis(2-ethylhexoxy)-1,4-dioxobutane-2-sulfonate solution and transferred to a round bottom flask. The THF was removed by rotoevaporation. The resulting yellow syrup was dissolved in 10 mL of acetone and then rotoevaporated to remove solvent. The sample was then placed under vacuum to remove residual acetone.

The product, erlotinib 1,4-bis(2-ethylhexoxy)-1,4-dioxobutane-2-sulfonate (erlotinib salt form), was a clear syrup at room temperature. A 50 mg sample of the erlotinib salt form, together with 150 mg of ethylcellulose (ETHOCEL® Std. 4) were dissolved in 5 mL methylene chloride to form an organic solution. Next, 2 mg of sodium glycocholate (a surfactant) was dissolved in 20 mL deionized water to form an aqueous solution.

The organic solution was then poured into the aqueous solution and emulsified for 3 min using a Kinematica Polytron 3100 rotor/stator (Kinematica AG, Lucerne, Switzerland) at 10,000 rpm (high-shear mixing). The solution was further emulsified using a Microfluidizer (Microfluidics [Newton, MA] model M-110S F12Y with ice bath and cooling coil), for 4 minutes at 100 psig (high-pressure homogenization). The methylene chloride was removed from the emulsion using a rotary evaporator at 35°C, resulting in an aqueous suspension of nanoparticles, with a composition mass ratio of 25:75 erlotinib salt form:ethylcellulose, in the sodium glycocholate aqueous suspension.

### Light Scattering Analysis

The particle size of the nanoparticles in the aqueous suspension was determined using dynamic light scattering (DLS) as follows. First, the aqueous suspension was filtered using a 1 µm glass membrane filter (Pall [Ann Arbor, MI] glass microfiber syringe filter), and poured into a cuvette. Dynamic light-scattering was measured using a Brookhaven Instruments (Holtsville, NY) BI-200SM particle size analyzer with a BI-9000AT correlator. The sums of exponentials from the autocorrelation functions were analyzed using CONTIN software to extract size distributions from the samples. The cumulant diameter of the nanoparticles in suspension was 212 nm. After storage at room temperature for one day, the particle size of the nanoparticles was determined to be 211 nm using the same method.

### Filter Potency

A filter potency test was used to characterize the nanoparticles of Example 1. A 100 µL sample of the aqueous nanoparticle suspension of Example 1 was added to 1 mL methanol, and the concentration of active agent in solution was analyzed by HPLC. HPLC was performed using a Zorbax® SB-C18, 4.6x150 mm, 5 µm column at 30°C, and a mobile phase comprising 20 mM ammonium acetate/acetonitrile (43/57 vol/vol). The flow rate was 1.0 mL/min, 10 µL of each solution was injected, and the absorbance was measured at 332 nm. The filter potency test was also performed after storage at room temperature for one day.

Potencies of the nanoparticle suspensions are shown in Table 1. These results indicate that the nanoparticles in suspension remained small and unagglomerated.

**Table 1.**

| Example | Filter Potency (mg/mL) | Particle Size (nm) |
|---|---|---|
| Initial | 1.69 | 212 |
| After 1 day storage | 1.71 | 211 |

### Example 2

Adapalene free acid (200.0 mg), having a pKa of 4.2, is dissolved in 100 mL THF. To this solution, N-benzyl-2-phenylethan-1-amine (102.5 mg) is added having a pKa of 9.4. The difference between the pKa of adapalene free acid with the counterion benethamine is 5.2. The resulting clear solution is stirred for several minutes and then roto-evaporated to remove solvent. The product, adapalene benethamine salt is obtained quantitatively. A 50 mg sample of the adapalene salt form, together with 150 mg of ethylcellulose (ETHOCEL® Std. 4) is dissolved in 5 mL methylene chloride to form an organic solution. Next, 2 mg of sodium glycocholate is dissolved in 20 mL deionized water to form an aqueous solution.

The organic solution is then poured into the aqueous solution and emulsified for 3 min using a Kinematica Polytron 3100 rotor/stator (Kinematica AG, Lucerne, Switzerland) at 10,000 rpm (high-shear mixing). The solution is further emulsified using a Microfluidizer (Microfluidics [Newton, MA] model M-110S F12Y with ice bath and cooling coil), for 4 minutes at 100 psig (high-pressure homogenization). The methylene chloride is removed from the emulsion using a rotary evaporator at 35°C, resulting in an aqueous suspension of nanoparticles, with a composition mass ratio of 25:75 adapaline benethamine salt form:ethylcellulose, in the sodium glycocholate aqueous suspension. Light scattering results shows that the nanoparticles have a size of less than 500 nm, using a similar process as used in Example 1.

## Claims

1. A composition comprising: nanoparticles having an average size of less than 1000 nm, said nanoparticles comprising
(a) a poorly aqueous soluble polymer;
(b) an ionizable active agent having a pKa; and
(c) a counterion having an opposite charge as the ionizable active agent for said active,
the counterion having a molecular weight of at least 200 Daltons, a Log P value of at least 2, and a pKa that is at least 1.5 units different than the pKa of said active,
wherein the active agent and the counterion are present in the form of a salt, wherein said active agent in said nanoparticles is substantially non-crystalline, wherein said active agent exists as a solid solution of active agent homogeneously distributed throughout said polymer, wherein said polymer, said ionizable active agent, and said counterion collectively constitute at least 80 wt% of said nanoparticles, wherein the molar ratio of said ionizable active agent to said counterion having an opposite charge is at least 0.8 and wherein said salt form in pure form has a melt temperature that is 10°C less than the melt temperature of the non-ionized form of said active in pure form.

2. The composition of claim 1 wherein the molar ratio of said ionizable active agent to said counterion having an opposite charge is at least 1.0.

3. The composition of any of the preceding claims, wherein said counterion is 1,4-bis(2-ethylhexoxy)-1 ,4-dioxobutane-2-sulfonate, cholesteryl sulfate, cholesteryl phosphate, tocopherol sulfate, tocopherol phosphate, 4,4'-methylenebis(3-hydroxy-2-naphthoate), 2,6-di-tertbutylnaphthalene-1-sulfonate, 2,6-di-tert-butylnaphthalene-1 ,5-disulfoate, 1,4-dicyclohexyloxy-1 ,4-dioxobutane-2-sulfonate, 1 ,4-diisopentyloxy-1 ,4-dioxobutane-2-sulfonate, 2-naphthalene sulfonate, or any combination thereof.

4. The composition of anyone of the preceding claims, wherein said counterion is cationic, and is benzathine, benethamine, hydrabamine, or a combination thereof.

5. The composition of any of the preceding claims, wherein said poorly aqueous soluble polymer is cellulose, ethylcellulose (EC), propylcellulose, butylcellulose, cellulose acetate (CA), cellulose propionate, cellulose butyrate, cellulose acetate butyrate (CAB), cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate (HPMCA), dextran acetate, dextran propionate, dextran acetate propionate, polyvinyl caprolactam, polyvinyl acetate, polyoxyethylene castor oils, polycaprolactam, polylactic acid, polyglycolic acid, poly(lactic-glycolic)acid, hydroxypropyl methyl cellulose acetate succinate (HPMCAS), hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1, poly(methacrylic acid-co-methyl methacrylate) 1 :2, poly(methacrylic acid-co-ethyl acrylate) 1:1, poloxamers, poly(ethylene oxide-b-£-caprolactone), poly(£-caprolactone-b-ethylene glycol), poly(ethylene oxide-b-lactide), poly(lactide-bethylene glycol), poly(ethylene oxide-b-glycolide), poly(glycolide-b-ethylene glycol), poly(ethylene oxide-b-lactide-co-glycolide), poly(lactide-coglycolide-b-ethylene glycol), or any mixture thereof.

6. The composition of any of the preceding claims, wherein said poorly aqueous soluble polymer is ethylcellulose (EC), cellulose acetate (CA), cellulose acetate butyrate (CAB), polylactic acid, polyglycolic acid, poly(lactic-glycolic)acid, or any mixture thereof.

7. The composition of any of the preceding claims, wherein said poorly aqueous soluble polymer is hydroxypropyl methyl cellulose propionate succinate, hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, carboxymethyl ethylcellulose (CMEC), hydroxypropyl methylcellulose acetate phthalate (HPMCAP), hydroxypropyl methylcellulose propionate phthalate, hydroxypropyl methylcellulose acetate trimellitate (HPMCAT), carboxymethyl cellulose acetate butyrate (CMCAB), hydroxypropyl methylcellulose propionate trimellitate, cellulose acetate succinate (CAS), methyl cellulose acetate succinate (MCAS), dextran acetate succinate, dextran propionate succinate, dextran acetate propionate succinate, poly(methacrylic acid-co-methyl methacrylate) 1:1 (e.g., Eudragit@ L 100, Evonik Industries AG), poly(methacrylic acid-co-methyl methacrylate) 1 :2 (e.g., Eudragit@ S100), poly(methacrylic acid-co-ethyl acrylate) 1:1 (e.g., Eudragit@ L 100-55), or any mixture thereof.

8. The composition of any of the preceding claims, wherein the composition consists essentially of the poorly aqueous soluble polymer, the ionizable active agent, and the counterion having an opposite charge as the ionizable active agent.

9. The composition of any of the preceding claims, wherein the composition consists of the poorly aqueous soluble polymer, the ionizable active agent, and the counterion having an opposite charge as the ionizable active agent.

10. A process of making the composition of any of the preceding claims, comprising:
(a) forming an organic solution comprising an organic solvent, a salt of an active agent and a counterion having an opposite charge as the ionizable active agent, and a poorly aqueous soluble polymer, wherein the molar ratio of said ionizable active agent to said counterion having an opposite charge is at least 0.8, and wherein said salt of a active agent and counterion in pure form has a melt temperature that is 10°C less than the melt temperature of the non-ionized form of said active in pure form;
(b) forming an aqueous solution, wherein said active agent, said counterion, and said polymer are poorly soluble in said aqueous solution;
(c) mixing said organic solution with said aqueous solution to form a first mixture;
(d) removing at least a portion of said organic solvent from said first mixture to form a suspension comprising said nanoparticles and said aqueous solution, wherein said nanoparticles have a size of less than 1000 nm, and wherein said active agent, said counterion, and said polymer collectively constitute at least 80 wt% of said nanoparticles.

11. The process of claim 10, wherein the molar ratio of said ionizable active agent to said counterion having an opposite charge is at least 1.0.

12. The process of any of the preceding claims, wherein the process is an emulsification process, and the organic solvent is immiscible with the aqueous solution.

13. The process of any of the preceding claims, wherein the process is a precipitation process and the organic solvent is miscible with the aqueous solution.

14. The process of any of the preceding claims, wherein said organic solvent is removed by spray drying, evaporation, extraction, diafiltration, pervaporation, vapor permeation, distillation, filtration, or any combination thereof.

15. The composition of any of the preceding claims, 1 to 9 that is formulated into a dosage form.

## Patentansprüche

1. Zusammensetzung, die Nanoteilchen mit einer durchschnittlichen Größe von weniger als 1000 nm umfassen, wobei die Nanoteilchen Folgendes umfassen:
(a) ein schlecht wasserlösliches Polymer;
(b) einen ionisierbaren Wirkstoff, der einen pKa-Wert aufweist; und
(c) ein Gegenion mit einer dem ionisierbaren Wirkstoff entgegengesetzten Ladung für den Wirkstoff, wobei das Gegenion ein Molekulargewicht von mindestens 200 Dalton, einen Log-P-Wert von mindestens 2 und einen pKa-Wert, der sich um mindestens 1,5 Einheiten von dem pKa-Wert des Wirkstoffs unterscheidet, aufweist,
wobei der Wirkstoff und das Gegenion in Form eines Salzes vorliegen, wobei der Wirkstoff in den Nanoteilchen weitgehend nicht kristallin ist, wobei der Wirkstoff als feste Lösung von in dem gesamten Polymer homogen verteiltem Wirkstoff vorhanden ist, wobei das Polymer, der ionisierbare Wirkstoff und das Gegenion zusammen mindestens 80Gew.-% der Nanoteilchen ausmachen, wobei das Molverhältnis von dem ionisierbaren Wirkstoff zu dem Gegenion mit entgegengesetzter Ladung mindestens 0,8 beträgt und wobei die Salzform in Reinform eine Schmelztemperatur aufweist, die 10 °C unter der Schmelztemperatur der nicht ionisierten Form des Wirkstoffs in Reinform liegt.

2. Zusammensetzung nach Anspruch 1, wobei das Molverhältnis von dem ionisierbaren Wirkstoff zu dem Gegenion mit entgegengesetzter Ladung mindestens 1,0 beträgt.

3. Zusammensetzung nach einem der vorhergehenden Ansprüche, wobei es sich bei dem Gegenion um 1,4-Bis(2-ethylhexoxy)-1,4-dioxobutan-2-sulfonat, Cholesterylsulfat, Cholesterylphosphat, Tocopherolsulfat, Tocopherolphosphat, 4,4'-Methylenbis(3-hydroxy-2-naphthoat), 2,6-Di-tert-butylnaphthalin-1-sulfonat, 2,6-Di-tert-butylnaphthalin-1,5-disulfoat, 1,4-Dicyclohexyloxy-1,4-dioxobutan-2-sulfonat, 1,4-Diisopentyloxy-1,4-dioxobutan-2-sulfonat, 2-Naphthalinsulfonat oder eine beliebige Kombination davon handelt.

4. Zusammensetzung nach einem der vorhergehenden Ansprüche, wobei es sich bei dem Gegenion um ein Kation und um Benzathin, Benethamin, Hydrabamin oder eine Kombination davon handelt.

5. Zusammensetzung nach einem der vorhergehenden Ansprüche, wobei es sich bei dem schlecht wasserlöslichen Polymer um Cellulose, Ethylcellulose (EC), Propylcellulose, Butylcellulose, Celluloseacetat (CA), Cellulosepropionat, Cellulosebutyrat, Celluloseacetatbutyrat (CAB), Celluloseacetatpropionat, Methylcelluloseacetat, Methylcellulosepropionat, Methylcellulosebutyrat, Ethylcelluloseacetat, Ethylcellulosepropionat, Ethylcelluloseabutyrat, Hydroxypropylmethylcelluloseacetat (HPMCA), Dextranacetat, Dextranpropionat, Dextranacetatpropionat, Polyvinylcaprolactam, Polyvinylacetat, Polyoxyethylen-Rizinusöle, Polycaprolactam, Polymilchsäure, Polyglykolsäure, Poly(milchglykolsäure, Hydroxypropylmethylcelluloseacetatsuccinat (HPMCAS), Hydroxypropylmethylcellulosepropionatsuccinat, Hydroxypropylmethylcellulosephthalat (HPMCP), Celluloseacetatphthalat (CAP), Celluloseacetattrimellitat (CAT), Methylcelluloseacetatphthalat, Hydroxypropylcelluloseacetatphthalat, Celluloseacetatterephthalat, Celluloseacetatisophthalat, Carboxymethylethylcellulose (CMEC), Hydroxypropylmethylcelluloseacetatphthalat (HPMCAP), Hydroxypropylmethylcellulosepropionatphthalat, Hydroxypropylmethylcelluloseacetattrimellitat (HPMCAT), Carboxymethylcelluloseacetatbutyrat (CMCAB), Hydroxypropylmethylcellulosepropionattrimellitat, Celluloseacetatsuccinat (CAS), Methylcelluloseacetatsuccinat (MCAS), Dextranacetatsuccinat, Dextranpropionatsuccinat, Dextranacetatpropionatsuccinat, Poly(methacrylsäure-co-methylmethacrylat) 1:1, Poly(methacrylsäure-co-methylmethacrylat) 1:2, Poly(methacrylsäure-co-ethylacrylat) 1:1, Poloxamere, Poly(ethylenoxid-b-£-caprolacton), Poly(£-caprolacton-b-ethylenglykol), Poly(ethylenoxid-b-lactid), Poly(lactid-b-ethylenglykol), Poly(ethylenoxid-b-glykolid), Poly(glykolid-b-ethylenglykol), Poly(ethylenoxid-b-lactid-co-glykolid), Poly(lactid-co-glykolid-b-ethylenglykol) oder eine beliebige Mischung davon handelt.

6. Zusammensetzung nach einem der vorhergehenden Ansprüche, wobei es sich bei dem schlecht wasserlöslichen Polymer um Ethylcellulose (EC), Celluloseacetat (CA), Celluloseacetatbutyrat (CAB), Polymilchsäure, Polyglykolsäure, Poly(milchglykolsäure) oder eine beliebige Mischung davon handelt.

7. Zusammensetzung nach einem der vorhergehenden Ansprüche, wobei es sich bei dem schlecht wasserlöslichen Polymer um Hydroxypropylmethylcellulosepropionatsuccinat, Hydroxypropylmethylcellulosephthalat (HPMCP), Celluloseacetatphthalat (CAP), Celluloseacetattrimellitat (CAT), Methylcelluloseacetatphthalat, Hydroxypropylcelluloseacetatphthalat, Celluloseacetatterephthalat, Celluloseacetatisophthalat, Carboxymethylethylcellulose (CMEC), Hydroxypropylmethylcelluloseacetatphthalat (HPMCAP), Hydroxypropylmethylcellulosepropionatphthalat, Hydroxypropylmethylcelluloseacetattrimellitat (HPMCAT), Carboxymethylcelluloseacetatbutyrat (CMCAB), Hydroxypropylmethylcellulosepropionattrimellitat, Celluloseacetatsuccinat (CAS), Methylcelluloseacetatsuccinat (MCAS), Dextranacetatsuccinat, Dextranpropionatsuccinat, Dextranacetatpropionatsuccinat, Poly(methacrylsäure-co-methylmethacrylat) 1:1 (z. B. Eudragit@ L100, Evonik Industries AG), Poly(methacrylsäure-co-methylmethacrylat) 1:2 (z. B. Eudragit@ S100), Poly(methacrylsäure-co-ethylacrylat) 1:1 (z. B. Eudragit@ L100-55) oder eine beliebige Mischung davon handelt.

8. Zusammensetzung nach einem der vorhergehenden Ansprüche, wobei die Zusammensetzung im Wesentlichen aus dem schlecht wasserlöslichen Polymer, dem ionisierbaren Wirkstoff und dem Gegenion mit dem ionisierbaren Wirkstoff entgegengesetzter Ladung besteht.

9. Zusammensetzung nach einem der vorhergehenden Ansprüche, wobei die Zusammensetzung aus dem schlecht wasserlöslichen Polymer, dem ionisierbaren Wirkstoff und dem Gegenion mit dem ionisierbaren Wirkstoff entgegengesetzter Ladung besteht.

10. Verfahren zur Herstellung der Zusammensetzung nach einem der vorhergehenden Ansprüche, das Folgendes umfasst:
(a) Bilden einer organischen Lösung, die ein organisches Lösungsmittel, ein Salz von einem Wirkstoff und ein Gegenion mit dem ionisierbaren Wirkstoff entgegengesetzter Ladung und ein schlecht wasserlösliches Polymer umfasst, wobei das Molverhältnis des ionisierbaren Wirkstoffs zu dem Gegenion mit entgegengesetzter Ladung mindestens 0,8 beträgt und wobei das Salz von einem Wirkstoff und Gegenion in Reinform eine Schmelztemperatur aufweist, die 10 °C unter der Schmelztemperatur der nicht ionisierten Form des Wirkstoffs in Reinform liegt;
(b) Bilden einer wässrigen Lösung, wobei der Wirkstoff, das Gegenion und das Polymer in der wässrigen Lösung schlecht löslich sind;
(c) Mischen der organischen Lösung mit der wässrigen Lösung zur Bildung einer ersten Mischung;
(d) Entfernen von zumindest einem Teil des organischen Lösungsmittels aus der ersten Mischung zur Bildung einer die Nanoteilchen und die wässrige Lösung umfassenden Suspension, wobei die Nanoteilchen eine Größe von weniger als 1000 nm aufweisen und wobei der Wirkstoff, das Gegenion und das Polymer zusammen mindestens 80 Gew.-% der Nanoteilchen ausmachen.

11. Verfahren nach Anspruch 10, wobei das Molverhältnis von dem ionisierbaren Wirkstoff zu dem Gegenion mit entgegengesetzter Ladung mindestens 1,0 beträgt.

12. Verfahren nach einem der vorhergehenden Ansprüche, wobei es sich bei dem Verfahren um ein Emulgierverfahren handelt und das organische Lösungsmittel mit der wässrigen Lösung nicht mischbar ist.

13. Verfahren nach einem der vorhergehenden Ansprüche, wobei es sich bei dem Verfahren um ein Fällungsverfahren handelt und das organische Lösungsmittel mit der wässrigen Lösung mischbar ist.

14. Verfahren nach einem der vorhergehenden Ansprüche, wobei das organische Lösungsmittel durch Sprühtrocknung, Verdampfung, Extraktion, Diafiltration, Pervaporation, Dampfpermeation, Destillation, Filtration oder eine beliebige Kombination davon entfernt wird.

15. Zusammensetzung nach einem der vorhergehenden Ansprüche 1 bis 9, die als Darreichungsform formuliert ist.

## Revendications

1. Composition comprenant des nanoparticules ayant une taille moyenne inférieure à 1000 nm, lesdites nanoparticules comprenant :
(a) un polymère peu soluble en milieu aqueux ;
(b) un principe actif ionisable ayant une valeur pKa ; et
(c) un contre-ion pour ledit principe actif ayant une charge opposée à celle du principe actif ionisable, le contre-ion ayant une masse moléculaire d'au moins 200 daltons, une valeur log P d'au moins 2, et une valeur pKa qui est d'au moins 1,5 unité différente de la valeur pKa dudit principe actif,
dans laquelle le principe actif et le contre-ion sont présents sous la forme d'un sel, dans laquelle ledit principe actif dans lesdites nanoparticules est sensiblement non cristallin, dans laquelle ledit principe actif est présent sous la forme d'une solution solide de principe actif répartie de façon homogène dans tout ledit polymère, dans laquelle ledit polymère, ledit principe actif ionisable, et ledit contre-ion constituent collectivement au moins 80 % en poids desdites nanoparticules, dans laquelle le rapport molaire dudit principe actif ionisable contre ledit contre-ion ayant une charge opposée est d'au moins 0,8, et dans laquelle ladite forme saline sous forme pure a une température de fusion qui est de 10 °C inférieure à la température de fusion de la forme non ionisée dudit principe actif sous forme pure.

2. Composition selon la revendication 1, dans laquelle le rapport molaire dudit principe actif ionisable contre ledit contre-ion ayant une charge opposée est d'au moins 1,0.

3. Composition selon l'une quelconque des revendications précédentes, dans laquelle ledit contre-ion est le 1,4-bis(2-éthylhexoxy)-1,4-dioxobutane-2-sulfonate, le sulfate de cholestéryle, le phosphate de cholestéryle, le sulfate de tocophérol, le phosphate de tocophérol, le 4,4'-méthylènebis(3-hydroxy-2-naphtoate), le 2,6-di-tert-butylnaphtalène-1-sulfonate, le 2,6-di-tert-butylnaphtalène-1,5-disulfoate, le 1,4-dicyclohexyloxy-1,4-dioxobutane-2-sulfonate, le 1,4-diisopentyloxy-1,4-dioxobutane-2-sulfonate, le 2-naphtalènesulfonate, ou n'importe quelle combinaison de ceux-ci.

4. Composition selon l'une quelconque des revendications précédentes, dans laquelle ledit contre-ion est cationique, et est la benzathine, la bénéthamine, l'hydrabamine, ou une combinaison de celles-ci.

5. Composition selon l'une quelconque des revendications précédentes, dans laquelle ledit polymère peu soluble en milieu aqueux est la cellulose, l'éthylcellulose (EC), la propylcellulose, la butylcellulose, l'acétate de cellulose (CA), le propionate de cellulose, le butyrate de cellulose, l'acéto-butyrate de cellulose (CAB), l'acéto-propionate de cellulose, l'acétate de méthylcellulose, le propionate de méthylcellulose, le butyrate de méthylcellulose, l'acétate d'éthylcellulose, le propionate d'éthylcellulose, le butyrate d'éthylcellulose, l'acétate d'hydroxypropylméthylcellulose (HPMCA), l'acétate de dextrane, le propionate de dextrane, l'acéto-propionate de dextrane, le polyvinylcaprolactame, l'acétate de polyvinyle, des huiles de ricin polyoxyéthylénées, le polycaprolactame, l'acide polylactique, l'acide polyglycolique, l'acide poly(lactique-glycolique), l'acéto-succinate d'hydroxypropylméthylcellulose (HPMCAS), le propionate-succinate d'hydroxypropylméthylcellulose, le phtalate d'hydroxypropylméthylcellulose (HPMCP), l'acéto-phtalate de cellulose (CAP), l'acéto-trimellitate de cellulose (CAT), l'acéto-phtalate de méthylcellulose, l'acéto-phtalate d'hydroxypropylcellulose, l'acéto-téréphtalate de cellulose, l'acéto-isophtalate de cellulose, la carboxyméthyléthylcellulose (CMEC), l'acéto-phtalate d'hydroxypropylméthylcellulose (HPMCAP), le propionate-phtalate d'hydroxypropylméthylcellulose, l'acéto-trimellitate d'hydroxypropylméthylcellulose (HPMCAT), l'acéto-butyrate de carboxyméthylcellulose (CMCAB), le propionate-trimellitate d'hydroxypropylméthylcellulose, l'acéto-succinate de cellulose (CAS), l'acéto-succinate de méthylcellulose (MCAS), l'acéto-succinate de dextrane, le propionate-succinate de dextrane, l'acéto-propionate-succinate de dextrane, le poly(acide méthacrylique-co-méthacrylate de méthyle) 1:1, le poly(acide méthacrylique-co-méthacrylate de méthyle) 1:2, le poly(acide méthacrylique-co-acrylate d'éthyle) 1:1, des poloxamères, le poly(oxyde d'éthylène-b-£-caprolactone), le poly(£-caprolactone-b-éthylène glycol), le poly(oxyde d'éthylène-b-lactide), le poly(lactide-b-éthylène glycol), le poly(oxyde d'éthylène-b-glycolide), le poly(glycolide-b-éthylène glycol), le poly(oxyde d'éthylène-b-lactide-co-glycolide), le poly(lactide-co-glycolide-b-éthylène glycol), ou n'importe quel mélange de ceux-ci.

6. Composition selon l'une quelconque des revendications précédentes, dans laquelle ledit polymère peu soluble en milieu aqueux est l'éthylcellulose (EC), l'acétate de cellulose (CA), l'acéto-butyrate de cellulose (CAB), l'acide polylactique, l'acide polyglycolique, l'acide poly(lactique-glycolique), ou n'importe quel mélange de ceux-ci.

7. Composition selon l'une quelconque des revendications précédentes, dans laquelle ledit polymère peu soluble en milieu aqueux est le propionate-succinate d'hydroxypropylméthylcellulose, le phtalate d'hydroxypropylméthylcellulose (HPMCP), l'acéto-phtalate de cellulose (CAP), l'acéto-trimellitate de cellulose (CAT), l'acéto-phtalate de méthylcellulose, l'acéto-phtalate d'hydroxypropylcellulose, l'acéto-téréphtalate de cellulose, l'acéto-isophtalate de cellulose, la carboxyméthyléthylcellulose (CMEC), l'acéto-phtalate d'hydroxypropylméthylcellulose (HPMCAP), le propionate-phtalate d'hydroxypropylméthylcellulose, l'acéto-trimellitate d'hydroxypropylméthylcellulose (HPMCAT), l'acéto-butyrate de carboxyméthylcellulose (CMCAB), le propionate-trimellitate d'hydroxypropylméthylcellulose, l'acéto-succinate de cellulose (CAS), l'acéto-succinate de méthylcellulose (MCAS), l'acéto-succinate de dextrane, le propionate-succinate de dextrane, l'acéto- propionate-succinate de dextrane, le poly(acide méthacrylique-co-méthacrylate de méthyle) 1:1 (par exemple Eudragit© L 100, Evonik Industries AG), le poly(acide méthacrylique-co-méthacrylate de méthyle) 1:2 (par exemple Eudragit© S100), le poly(acide méthacrylique-co-acrylate d'éthyle) 1:1 (par exemple Eudragit© L 100-55), ou n'importe quel mélange de ceux-ci.

8. Composition selon l'une quelconque des revendications précédentes, la composition se composant essentiellement du polymère peu soluble en milieu aqueux, du principe actif ionisable, et du contre-ion ayant une charge opposée à celle du principe actif ionisable.

9. Composition selon l'une quelconque des revendications précédentes, la composition se composant du polymère peu soluble en milieu aqueux, du principe actif ionisable, et du contre-ion ayant une charge opposée à celle du principe actif ionisable.

10. Procédé de fabrication de la composition selon l'une quelconque des revendications précédentes, comprenant :
(a) la formation d'une solution organique comprenant un solvant organique, un sel d'un principe actif et d'un contre-ion ayant une charge opposée à celle du principe actif ionisable, et un polymère peu soluble en milieu aqueux, dans laquelle le rapport molaire dudit principe actif ionisable contre ledit contre-ion ayant une charge opposée est d'au moins 0,8, et dans laquelle ledit sel d'un principe actif et d'un contre-ion sous forme pure a une température de fusion qui est de 10 °C inférieure à la température de fusion de la forme non ionisée dudit principe actif sous forme pure ;
(b) la formation d'une solution aqueuse, ledit principe actif, ledit contre-ion et ledit polymère étant peu solubles dans ladite solution aqueuse ;
(c) le mélange de ladite solution organique avec ladite solution aqueuse pour former un premier mélange ;
(d) le retrait d'au moins une partie dudit solvant organique dudit premier mélange pour former une suspension comprenant lesdites nanoparticules et ladite solution aqueuse, lesdites nanoparticules ayant une taille inférieure à 1000 nm, et ledit principe actif, ledit contre-ion et ledit polymère constituant collectivement au moins 80 % en poids desdites nanoparticules.

11. Procédé selon la revendication 10, dans lequel le rapport molaire dudit principe actif ionisable contre ledit contre-ion ayant une charge opposée est d'au moins 1,0.

12. Procédé selon l'une quelconque des revendications précédentes, le procédé étant un procédé d'émulsification, et le solvant organique étant immiscible avec la solution aqueuse.

13. Procédé selon l'une quelconque des revendications précédentes, le procédé étant un procédé de précipitation, et le solvant organique étant miscible avec la solution aqueuse.

14. Procédé selon l'une quelconque des revendications précédentes, dans lequel ledit solvant organique est retiré par séchage par atomisation, évaporation, extraction, diafiltration, pervaporation, perméation de vapeur, distillation, filtration, ou n'importe quelle combinaison de ceux-ci.

15. Composition selon l'une quelconque des revendications précédentes 1 à 9, laquelle est formulée en une forme galénique.
